Europäisches Patentamt

**European Patent Office**

Office européen des brevets

(19)

(11) Numéro de publication: **0 254 621**
**B1**

(12) **FASCICULE DE BREVET EUROPEEN**

(45) Date de publication du fascicule du brevet:
11.04.90

(51) Int. Cl.⁴: **A61L 9/01**

(21) Numéro de dépôt: 87401565.4

(22) Date de dépôt: 03.07.87

(54) **Liquide désodorisant ininflammable.**

(30) Priorité: 21.07.86 FR 8610555

(43) Date de publication de la demande:
27.01.88 Bulletin 88/4

(45) Mention de la délivrance du brevet:
11.04.90 Bulletin 90/15

(84) Etats contractants désignés:
BE CH DE FR GR IT LI LU NL SE

(56) Documents cités:

(73) Titulaire: **Reckitt & Colman S.A., 15, rue Ampère,
F-91301 Massy Cédex(FR)**

(72) Inventeur: **Dumas, Sylvie, 27 rue des Neufs Soleils
Bâtiment E, F-6300 Clermont-Ferrand(FR)**

(74) Mandataire: **Rinuy, Santarelli, 14, avenue de la Grande
Armée, F-75017 Paris(FR)**

ACTORUM AG

**Description**

L'invention concerne des compositions à utiliser dans des dispositifs de désodorisation de l'air pour éliminer des odeurs déplaisantes et/ou pour produire une atmosphère parfumée, par exemple à l'intérieur d'une pièce.

Divers types de dispositifs de désodorisation de l'air (désodoriseurs de l'air) existent sur le marché ; ils ont une action continue ou non et sont basés sur des types très différents de technologie de diffusion et de technologie de support, tels que des aérosols, des gels, des tampons de cellulose imprégnés, l'évaporation électrique et les évaporateurs à mèche à liquide.

Les évaporateurs à mèche à liquide (c'est-à-dire les désodoriseurs d'air comprenant un réservoir de liquide dans lequel plonge une mèche afin de transférer le liquide vers l'extérieur par évaporation) sont particulièrement efficaces pour une évaporation constante pendant une période de temps. Un autre avantage est qu'on peut voir aisément lorsque la composition désodorisante arrive à épuisement.

La majorité de ces liquides sont des solutions aqueuses (pour des raisons économiques) qui contiennent une petite quantité de parfum. Pour dissoudre les parfums dans de telles solutions et obtenir une composition homogène, on a utilisé des surfactants : les surfactants non volatils tendent à s'accumuler sur la mèche, empêchant ainsi la montée du liquide le long de la mèche et finissant par empêcher complètement l'évaporation.

Pour assurer une longue durée d'action, il faut une grande quantité de liquide, ce qui, par suite, nécessite un grand récipient. Ceci est inacceptable pour le consommateur pour des raisons d'aspect esthétique et de coût.

La puissance du désodorisant est accrue si l'on ajoute davantage de parfum : cependant, pour maintenir l'homogénéité, il est alors nécessaire d'ajouter de plus grandes quantités de surfactants, ce qui peut conduire à un blocage prématuré de la mèche.

Une solution à ce problème consiste à utiliser des formules à base de solvant organique qui permettent la dissolution d'une plus grande quantité de parfum. Par conséquent, il n'est besoin que de petites quantités de surfactant, voire d'aucun surfactant, et les volumes d'ensemble peuvent être réduits sans que l'efficacité du produit en soit affectée. Le solvant assume la fonction de support pour le parfum. Il aide le parfum à monter le long de la mèche et à s'évaporer à la surface.

Les formulations de solvant contiennent généralement de l'eau pour des raisons d'économie. Cependant, ceci n'élimine généralement pas un inconvénient que de telles formulations peuvent présenter, à savoir l'inflammabilité.

Il est cependant possible de réaliser le dispositif de désodorisation de l'air de façon que la surface d'évaporation soit isolée de tout contact avec des flammes. En général, ceci nécessite de placer une grille protectrice au-dessus du récipient : ceci peut nuire à la qualité de l'évaporation. La demande porte sur un dispositif compact de désodorisation de l'air à base de solvants, fonctionnant de façon satisfaisante.

On a découvert qu'en utilisant un co-solvant particulier dans un liquide, on inhibe ou on supprime totalement l'inflammabilité des vapeurs lorsque la surface d'évaporation est en contact avec des flammes. Ceci évite d'avoir à utiliser une grille protectrice, de sorte que le dispositif est moins coûteux à produire et plus plaisant du point de vue esthétique. Un tel cosolvant est un phosphonate -en particulier le méthylphosphonate de diméthyle (DMMP) qui est un liquide que l'on utilisait jusqu'à présent pour ses propriétés d'ignifugation dans des matières plastiques et des polyuréthannes (voir, par exemple, le brevet européen publié sous le N° 108713-A (Ciba-Geigy AG) et le brevet de la RFA N° 27 40 589-A (Stauffer Chemical Co.)).

On a à présent découvert que l'introduction d'une petite quantité de DMMP dans une composition liquide homogène de désodorisation de l'air à base de solvant organique peut rendre le produit ininflammable.

Conformément à l'invention, il est proposé une composition homogène non inflammable de désodorisation de l'air comprenant 3 à 15 % de méthylphosphonate de diméthyle, 1 à 12 % de parfum, 40 à 65 % de solvant organique et 15 à 50 % d'eau (les pourcentages étant donnés en poids de la composition totale).

Selon un aspect de l'invention, le méthylphosphonate de diméthyle est compris entre 4 et 8 %, le parfum entre 7 et 10 %, le solvant organique entre 45 et 50 % et l'eau entre 37 et 42 %.

Des solvants organiques convenables sont des alcools (par exemple méthanol, éthanol, 1-propanol et 2-propanol), des glycols (par exemple, 1,2-éthanediol et 1,2-propanediol), des mono- et des diéthers de glycols (par exemple éthers méthylique et éthylique de 1,2-éthanediol ou 1,2-propanediol) et des mélanges de ces substances. La quantité d'eau présente doit évidemment être telle que le parfum et le solvant sont miscibles avec elle.

Selon un autre aspect de l'invention, la composition comprend 4 à 8 % de méthylphosphonate de diméthyle, 7 à 10 % de parfum, 45 à 50 % d'éther monoéthylique de 1,2-propanediol et 37 à 42 % d'eau.

La quantité de DMMP nécessaire pour rendre la composition ininflammable dépend de l'inflammabilité à la fois du solvant organique et du parfum et des proportions de solvant organique, de parfum et d'eau présentes. Des compositions dont le rapport du poids total du solvant organique et du parfum au poids du DMMP est compris entre 3:1 et 20:1 se sont avérées particulièrement satisfaisantes.

Un autre avantage du DMMP est qu'il est sans odeur et qu'il n'affecte donc pas les propriétés désodorisantes de solution.

Il convient de souligner qu'il n'est pas néeessaire que la composition de désodorisation soit inflammable directement, même sans l'addition de DMMP. Le problème de l'inflammabilité se pose lorsque la composition est utilisée dans un évaporateur à mèche à liquide dans lequel de petites quantités de la composition sont distribuées à l'atmosphère. Une analogie utile est celle de la bougie dont la cire n'est pas directement inflammable mais est capable d'entretenir une flamme par l'intermédiaire d'une mèche.

Pour démontrer l'ininflammabilité d'un système liquide/mèche, on peut placer 40 ml du liquide dans un bécher de 50 ml, d'un diamètre de moins de 46 mm. Le mécanisme d'évaporation, consistant en une surface évaporante (un disque de 50 mm de diamètre réalisé en coton ou en cellulose) et une mèche (de coton ou de cellulose), fixée au milieu, est placé sur le bécher, la mèche plongeant dans le liquide. On laisse celui-ci s'évaporer pendant 24 heures, après quoi une allumette allumée est posée sur la surface évaporante. Ceci est répété trois fois. En l'absence de flamme, lorsque l'allumette est retirée, le produit peut être considéré comme étant ininflammable. Cependant, si les vapeurs brûlent après le retrait de l'allumette, le produit est jugé comme étant inflammable.

L'invention est illustrée par les exemples montrés dans le tableau. Ce dernier contient des exemples comparatifs dans lesquels le DMMP a été supprimé : les exemples 1, 4, 7, 10, 12 (sans DMMP) correspondent aux exemples 2, 5, 8, 11, 13 (avec DMMP) par le fait que les composants sont présents dans des proportions identiques entre eux. Il convient de noter que les exemples 3, 6 et 9 sont uniquement des témoins et non des compositions de désodorisation de l'air.

| Example | 1 | 2 | 3 | 4 | 5 | 6 | 7 | 8 | 9 | 10 | 11 | 12 | 13 |
|---|---|---|---|---|---|---|---|---|---|---|---|---|---|
| Composition (% en poids) | | | | | | | | | | | | | |
| Ethanol | 53,3 | 48 | — | — | — | — | — | — | — | — | — | — | — |
| 2-méthoxyéthanol | — | — | 100 | 65,2 | 57,4 | — | — | — | — | — | — | — | — |
| 2-éthoxyéthanol | — | — | — | — | — | 100 | 60 | 57 | — | — | — | — | — |
| Napsol PE$_1$* | — | — | — | — | — | — | — | — | 100 | 47,4 | 45 | 52,6 | 50 |
| Eau | 37,8 | 34 | — | 25,2 | 22,2 | — | 31,6 | 30 | — | 44,2 | 42 | 39,0 | 37 |
| Parfum** | 8,9 | 8 | — | 9,6 | 8,4 | — | 8,4 | 8 | — | 8,4 | 8 | 8,4 | 8 |
| DMMP | — | 10 | — | — | 12 | — | — | 5 | — | — | 5 | — | 5 |
| Rapport — (solvant + parfum): DMMP | | 5,6 | | | 5,5 | | | 13,0 | | | 10,6 | — | 11,6 |
| Inflammabilité | Oui | Non | Oui | Oui | Non | Oui | Oui | Non | Oui | Oui | Non | Oui | Non |

\* Ether mono-éthylique de 1,2-propanediol

\*\* Parfums utilisés: LF 11451 et 11453 (Bush Boake Allen)

Il ressort du tableau que l'addition de DMMP à une composition de désodorisation de l'air permet de supprimer efficacement l'inflammabilité de la composition.

Il est inutile d'indiquer que l'invention a été décrite uniquement à titre illustratif et non limitatif. Les proportions respectives de DMMP:parfum:solvant:eau peuvent être modifiées sans sortir du cadre de l'invention.

## Revendications

1. Composition homogène ininflammable de désodorisation de l'air, caractérisée en ce qu'elle comprend 3 à 15 % de méthylphosphonate de diméthyle, 1 à 12 % de parfum, 40 à 65 % de solvant organique convenable et 15 à 50 % d'eau, les pourcentages étant des pourcentages en poids de la composition totale.

2. Composition selon la revendication 1, caractérisée en ce que le méthylphosphonate de diméthyle est compris entre 4 et 8 %, le parfum entre 7 et 10 %, le solvant organique entre 45 et 50 % et l'eau entre 37 et 42 %, ces pourcentages étant des pourcentages en poids de la composition totale.

3. Composition selon l'une des revendications 1 ou 2, caractérisée en ce que le solvant organique comprend un alcool, un glycol ou un mono- ou diéther d'un glycol, ou un mélange de ces substances.

4. Composition selon la revendication 3, caractérisée en ce que le solvant organique comprend du méthanol, de l'éthanol, du 1-propanol, du 2-propanol, du 1,2-éthanediol, du 1,2-propanediol, ou un mono-

3

ou diéther de 1,2-éthanediol ou de 1,2-propanediol, ou des mélanges de ces substances.

5. Composition selon la revendication 4, caractérisée en ce que le mono- ou le diéther comprend un éther de méthyle ou d'éthyle, ou un mélange de ceux-ci.

6. Composition selon la revendication 4, caractérisée en ce que le solvant organique comprend de l'éthanol, du 2-méthoxyéthanol, du 2-éthoxyéthanol ou de l'éther mono-éthylique de 1,2-propanediol, ou des mélanges de ces substances.

7. Composition selon la revendication 2, caractérisée en ce que le solvant organique est de l'éther mono-éthylique de 1,2-propanediol.

8. Composition selon la revendication 1, caractérisée en ce que le rapport du poids total du solvant organique et du poids du parfum au poids du méthylphosphonate de diméthyle est compris entre 3:1 et 20:1.

**Claims**

1. A non-flammable homogeneous air deodorization composition, characterised in that it comprises 3 to 15% dimethyl methylphosphonate, 1 to 12% fragrance, 40 to 65% suitable organic solvent and 15 to 50% water (where percentages are percentage by weight of the total composition).

2. A composition according to claim 1, characterised in that the dimethyl methylphosphonate is in the range 4 to 8%, the fragrance is in the range 7 to 10%, the organic solvent is in the range 45 to 50% and the water is in the range 37 to 42% (where percentages are percentage by weight of the total composition).

3. A composition according to any one of claims 1 to 2, characterised in that the organic solvent comprises an alcohol, a glycol or a mono- or diether of a glycol, or a mixture thereof.

4. A composition according to claim 3, characterised in that the organic solvent comprises methanol, ethanol, 1-propanol, 2-propanol, 1, 2-ethanediol, 1, 2-propanediol, or a mono- or diether of 1, 2-ethanediol or 1, 2-propanediol, or mixtures thereof.

5. A composition according to claim 4, characterised in that the mono- or diether comprises a methyl or ethyl ether, or a mixture thereof.

6. A composition according to claim 4, characterised in that the organic solvent comprises ethanol, 2-methoxyethanol, 2-ethoxyethanol or 1, 2-propanediol monoethyl ether, or mixtures thereof.

7. A composition according to claim 2, characterised in that the organic solvent is 1, 2-propanediol monoethyl ether.

8. A composition according to claim 1, characterised in that the ratio of the total weight of the organic solvent plus the weight of fragrance to the weight of dimethyl methylphosphate is in the range 3:1 to 20:1.

**Patentansprüche**

1. Homogene nicht-entzündbare Zusammensetzung zur Geruchsbeseitigung bei Luft, dadurch gekennzeichnet, daß sie 3 bis 15% Dimethyl-methylphosphonat, 1 bis 12% Parfüm, 40 bis 65% verträgliches organisches Lösungsmittel und 15 bis 50% Wasser umfaßt, wobei die Prozentangaben Gewichtsprozente bezogen auf die Gesamt-Zusammensetzung sind.

2. Zusammensetzung nach Anspruch 1, dadurch gekennzeichnet, daß der Anteil des Dimethyl-methylphosphonats zwischen 4 und 8%, des Parfüms zwischen 7 und 10%, des organische Lösungsmittels zwischen 45 und 50% und des Wassers zwischen 37 und 42% beträgt, wobei diese Prozentangaben Gewichtsprozente bezogen auf die Gesamt-Zusammensetzung sind.

3. Zusammensetzung nach einem der Ansprüche 1 oder 2, dadurch gekennzeichnet, daß das organische Lösungsmittel einen Alkohol, ein Glykol oder einen Mono- oder Diäther eines Glykols oder ein Gemisch dieser Substanzen umfaßt.

4. Zusammensetzung nach Anspruch 3, dadurch gekennzeichnet, daß das organische Lösungsmittel Methanol, Äthanol, 1-Propanol, 2-Propanol, 1, 2-Äthandiol, 1, 2-Propandiol oder einen Mono- oder Diäther von 1, 2-Äthandiol oder von 1, 2-Propandiol oder Gemische dieser Substanzen umfaßt.

5. Zusammensetzung nach Anspruch 4, dadurch gekennzeichnet, daß der Mono- oder Diäther einen Methyl- oder Äthyläther oder ein Gemisch desselben umfaßt.

6. Zusammensetzung nach Anspruch 4, dadurch gekennzeichnet, daß das organische Lösungsmittel Äthanol, 2-Methoxyäthanol, 2-Äthoxyäthanol oder den Monoäthyläther von 1, 2-Propandiol oder Gemische dieser Substanzen umfaßt.

7. Zusammensetzung nach Anspruch 2, dadurch gekennzeichnet, daß das organische Lösungsmittel Monoäthyläther von 1, 2-Propandiol ist.

8. Zusammensetzung nach Anspruch 1, dadurch gekennzeichnet, daß das Verhältnis des Gesamtgewichts des organischen Lösungsmittels und des Gewichts des Parfüms zum Gewicht des Dimethyl-methylphosphonats zwischen 3:1 und 20:1 liegt.